(19) Europäisches Patentamt European Patent Office Office européen des brevets

(11) **EP 4 272 672 A1**

(12) **EUROPEAN PATENT APPLICATION**
published in accordance with Art. 153(4) EPC

(43) Date of publication:
**08.11.2023  Bulletin 2023/45**

(21) Application number: **21914573.7**

(22) Date of filing: **29.12.2021**

(51) International Patent Classification (IPC):
***A61B 18/12*** *(2006.01)*

(86) International application number:
**PCT/CN2021/142752**

(87) International publication number:
**WO 2022/143841 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **31.12.2020   CN 202011640891**

(71) Applicant: **Hangzhou Broncus Medical Co., Ltd.
Hangzhou, Zhejiang 310051 (CN)**

(72) Inventors:
• **CUI, Changjie**
  **Hangzhou, Zhejiang 310051 (CN)**
• **XU, Hong**
  **Hangzhou, Zhejiang 310051 (CN)**

(74) Representative: **Petraz, Gilberto Luigi et al
GLP S.r.l.
Viale Europa Unita, 171
33100 Udine (IT)**

(54)  **POWER ADJUSTMENT METHOD FOR MULTI-ELECTRODE RADIO FREQUENCY PROBE, AND RADIO FREQUENCY HOST**

(57)     A power adjustment method for multi-electrode radio frequency probe and a radio frequency host, wherein the power adjustment method for multi-electrode radio frequency probe includes: detecting physical characteristic data of a radio frequency operating object in real-time; controlling an injection pump to inject a liquid into the radio frequency operating object according to a preset injection flow velocity when the physical characteristic data exceeds the reference value range, so as to adjust the physical characteristic data to be within the reference value range; calculating a power adjustment value of a radio frequency host by means of a preset algorithm when the injection flow velocity of the injection pump reaches a limit value and the physical characteristic data exceeds the reference value range; adjusting a radio frequency output power according to the power adjustment value, which can improve the success rate of a radio frequency operation.

detecting physical characteristic data of a RF operating object in real-time when a RF energy emitted by a RF host according to a first power acts on the RF operating object through a RF probe, and comparing the physical characteristic data with a preset reference value range — S301

controlling an injection pump to inject a liquid into the RF operating object according to a preset injection flow velocity when the physical characteristic data exceeds the reference value range, so as to adjust the physical characteristic data to be within the reference value range — S302

calculating a power adjustment value of the RF host according to electrical parameters of electrodes of the multi-electrode RF probe when the injection flow velocity of the injection pump reaches a limit value and the physical characteristic data exceeds the reference value range — S303

adjusting a RF output power to a second power according to the first power and the power adjustment value and then outputting the second power — S304

FIG. 3

EP 4 272 672 A1

**Description**

## TECHNICAL FIELD

Embodiments of the present invention relate to the field of electronic technology, and in particular to a power adjustment method for multi-electrode radio frequency probe and a radio frequency host.

## BACKGROUND

Radio frequency (RF) technology is guided by images, wherein a RF probe enters an operating position of an operating object, and a RF host sends a RF energy which is accurately applied to the operating object for RF operation. During the RF operation process, it is necessary to ensure the operation effect and also pay attention to protecting the operating object and operator from damage and injure. When the power of the RF energy is too high, it is easy to cause damage to the operating object and operator.

In the prior art, in case of an application scenario of a RF probe with multiple electrodes, power setting can only be made for multiple electrodes as a whole, which is not precise enough and affects an effectiveness of the RF operation.

## SUMMARY

Embodiments of the present invention provides a power adjustment method for multi-electrode radio frequency probe and a radio frequency host, which can realize power setting according to electrical parameters of electrodes of the multi-electrode radio frequency probe, improving an accuracy of power control, and thus improving an effectiveness of operation.

On one aspect, an embodiment of the present invention provides a power adjustment method for multi-electrode radio frequency probe, including:

detecting physical characteristic data of a radio frequency operating object in real-time when a radio frequency energy emitted by a radio frequency host according to a first power acts on the radio frequency operating object through a multi-electrode radio frequency probe, and comparing the physical characteristic data with a preset reference value range;

controlling an injection pump to inject a liquid into the radio frequency operating object according to a preset injection flow velocity when the physical characteristic data exceeds the reference value range, so as to adjust the physical characteristic data to be within the reference value range;

calculating a power adjustment value of the radio frequency host according to electrical parameters of electrodes of the multi-electrode radio frequency probe when the injection flow velocity of the injection pump reaches a limit value and the physical characteristic data exceeds the reference value range;

adjusting a radio frequency output power to a second power according to the first power and the power adjustment value and then outputting the second power.

On another aspect, an embodiment of the present invention further provides a radio frequency host, including:

a detection module, configured for controlling an injection pump to inject a liquid into the radio frequency operating object according to a preset injection flow velocity when the physical characteristic data exceeds the reference value range, so as to adjust the physical characteristic data to be within the reference value range;

a control module, configured for controlling an injection pump to inject a liquid into the radio frequency operating object according to a preset injection flow velocity when the physical characteristic data exceeds the reference value range, so as to adjust the physical characteristic data to be within the reference value range;

the control module further configured for calculating a power adjustment value of the radio frequency host according to electrical parameters of electrodes of the multi-electrode radio frequency probe when the injection flow velocity of the injection pump reaches a limit value and the physical characteristic data exceeds the reference value range;

the control module further configured for adjusting a radio frequency output power to a second power according to the first power and the power adjustment value and then outputting the second power.

On another aspect, an embodiment of the present invention further provides a radio frequency host including a memory and a processor, the memory storing executable program codes thereon, the processor being electrically coupled to the memory, calling the executable program codes stored on the memory and executing the power adjustment method for multi-electrode radio frequency probe as described above.

From the above embodiments of the present invention, it can be seen that the injection pump is first controlled to inject the liquid into the radio frequency operating object according to the preset injection flow velocity when the physical characteristic data exceeds the reference value range, so as to adjust the physical characteristic data to be within the reference value range. If the physical characteristic data still exceeds the reference value range when the injection flow velocity reaches the limit value, the power adjustment value of the radio frequency host is then calculated according to the electrical parameters of the electrodes of the multi-electrode radio frequency probe, and the output power is adjusted according to the power adjustment value. By means of adding the electrical parameters of the electrodes into the calculation, an accuracy of power control adjustment can be improved, and an effectiveness of radio frequency operation can be improved.

## BRIEF DESCRIPTION OF THE DRAWINGS

In order to describe the technical solutions according to the embodiments of the present invention or in the prior art more clearly, the drawings needed to be used in the embodiments or in the prior art will be described briefly below. Apparently, the drawings in the following description show some embodiments of the present invention. Other drawings can be obtained by persons of ordinary skill in the art according to these drawings without creative efforts.

FIG. 1 is a schematic diagram showing an application scenario of a power adjustment method for multi-electrode radio frequency probe provided by an embodiment of the present invention.
FIG. 2 is a schematic diagram showing a connection between a multi-electrode radio frequency probe and a radio frequency host according to an embodiment of the present invention.
FIG. 3 is a flowchart of a power adjustment method for multi-electrode radio frequency probe provided by an embodiment of the present invention.
FIG. 4 is a flowchart of a power adjustment method for multi-electrode radio frequency probe provided by another embodiment of the present invention.
FIG. 5 is a schematic view of a radio frequency host provided by an embodiment of the present invention.
FIG. 6 is a schematic view of a radio frequency host provided by another embodiment of the present invention.
FIG. 7 is a schematic view of a hardware structure of a radio frequency host provided by an embodiment of the present invention.

## DESCRIPTION OF THE EMBODIMENTS

In order to make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present invention. All other embodiments obtained by ordinary persons skilled in the art according to the embodiments of the present invention without creative efforts shall fall within the protection scope of the present invention.

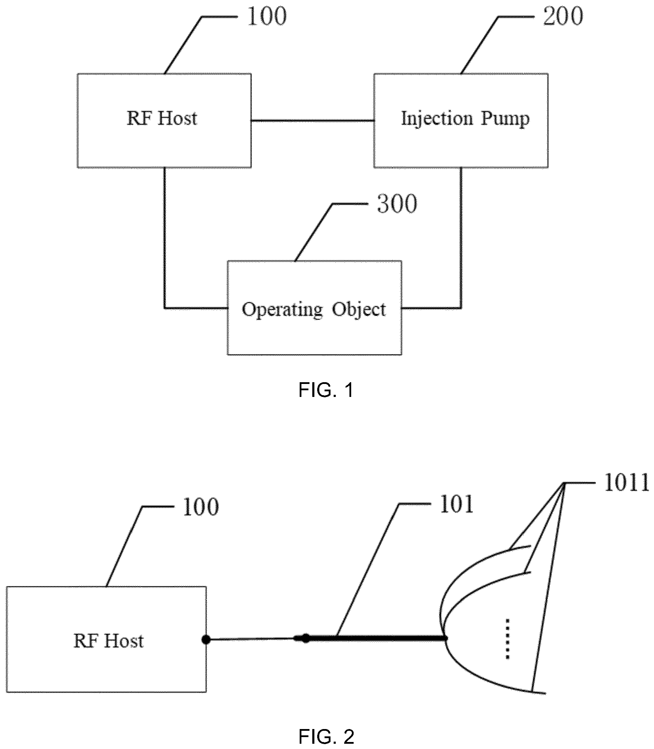

Referring to FIG. 1, which is a schematic diagram showing an application scenario of data adjustment in a radio frequency (RF) operation provided by an embodiment of the present invention. A power adjustment method of a multi-electrode RF probe is used to calculate a power adjustment value according to a PID (Proportional Integral Differential) algorithm when an event triggering power adjustment occurs during the RF operation, such as a temperature of a RF operating object exceeds a preset value. According to a first working power value before adjustment and the power adjustment value, a second working power value after adjustment is calculated, improving a success rate and an effectiveness of the RF operation. PID control algorithm is a control algorithm that combines proportion, integration and differentiation together. The essence of the PID control is to calculate according to functional relationships of proportion, integration, and differentiation according to an input deviation value, wherein a calculation result is used to control the output.

Specifically, an executing entity of the data adjustment method is a RF host, which may be devices such as RF ablation devices. As shown in FIG. 1, a RF host 100 and an injection pump 200 are connected to a RF operating object 300, respectively. The RF host 100 includes a multi-electrode RF probe. Further referring to FIG. 2, the multi-electrode RF probe 101 with multiple electrodes 1011 is connected to the RF host 100. The RF operating object 300 may be any object that needs to do a RF operation. For example, when the RF host 100 is a RF ablation device, the RF operating object may be an organism with mutated tissues within the body that needs to be ablated, and the ablation device emits a RF energy to ablate the mutated tissues.

During the RF operation, the RF host 100 emits the RF energy according to a first power through a RF generator. The first power may be preset, or may be a value matched with characteristics of the RF operation object 300 obtained from the Big data of the RF operation stored on the server. The RF energy acts on a designated position of the RF operation object 300 through the multi-electrode RF probe. During the RF operation process, physical characteristic data detected by a detection device of the RF host 100 will change as the shape and property of the designated position of the RF operating object 300 change. The injection pump 200 includes an injection device, which injects a liquid into the designated position (i.e. operating position) of the RF operating object 300 under the control of a control device of the injection pump 200. The physical characteristic of the operating position may be adjusted by adjusting an injection flow velocity of the liquid. The liquid is safe and harmless, such as physiological saline and the like. The RF host 100 and the injection pump 200 are connected to construct a RF operating system, wherein the RF host 100 acts as a host device of the RF operating system and the injection pump 200 acts as a slave device after being connected. The injection pump 200 itself loses control about an injection operation, and the control device of the RF host 100 controls the injection pump 200 to perform the injection operation.

Both the RF host 100 and the injection pump 200 include input interfaces, which may be connected to external removable memory, such as USB disk, or may be connected to external input devices, such as keyboard, mouse and etc., reading data from the removable memory, and acquiring user input data from the input devices. The RF host 100 further may be connected to a server through the network to obtain Big data from a database of the server, wherein the Big data comes from all RF hosts connected to the server, and includes various historical data related to the RF operation, such as a working power and a working time of the RF operation, a temperature and an impedance of the operating position of the RF operation object.

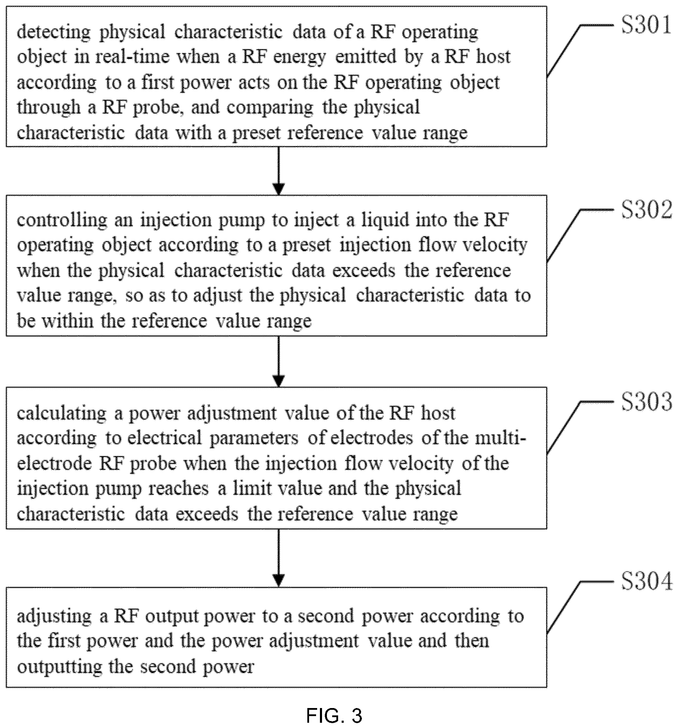

Referring to FIG. 3, which is a flowchart of a power adjustment method for multi-electrode RF probe provided by an embodiment of the present invention. The method can be applied to the RF host as shown in FIG. 1. As shown in FIG. 3, the method specifically includes:

Step S301: detecting physical characteristic data of a RF operating object in real-time when a RF energy emitted by a RF host according to a first power acts on the RF operating object through a multi-electrode RF probe, and comparing the physical characteristic data with a preset reference value range;

The multi-electrode RF probe includes multiple electrodes, which act on the designated position of the RF operating object simultaneously during the RF operation. There is no limit to the number of multiple electrodes, which may be 3, 4, 6, and etc., depending on the purpose of the RF operation, the nature of the RF operation object, and the characteristics of the designated position.

The physical characteristic data may be a temperature value of the RF operating object.

When the multi-electrode RF probe performs the RF operation on the RF operating object, the physical characteristic data of the RF operating object will change. The physical characteristic data is compared with the preset reference value range to determine whether the physical characteristic data exceeds the reference value range, for example, determine whether the temperature value of the RF operating object exceeds a preset temperature reference value range.

Step S302: controlling an injection pump to inject a liquid into the RF operating object according to a preset injection flow velocity when the physical characteristic data exceeds the reference value range, so as to adjust the physical characteristic data to be within the reference value range;

The reference value range is a numerical range with the minimum and maximum values. The minimum and maximum values may be obtained from historical RF operation data of all RF hosts in the network of the server, or from setting data input by the user into the RF host. The physical characteristic data exceeds the reference value range, that is, it is greater than the maximum value of the reference value range, or it is less than the minimum value of the reference value range.

When a control system of the RF host determines that the physical characteristic data of the RF operating object exceeds the reference value range, it sends a control instruction immediately or after a preset waiting time (such as 3 seconds) to a control system of the injection pump to control the injection pump to inject the liquid into the RF operating object according to the preset injection flow velocity, so as to adjust the physical characteristic data to be within the reference value range. The preset injection flow velocity is greater or less than the current injection flow velocity. Increasing the injection flow velocity can decrease the temperature value of the RF operating object, while decreasing the injection flow velocity can increase the temperature value of the RF operating object.

Step S303: calculating a power adjustment value of the RF host according to electrical parameters of electrodes of the multi-electrode RF probe when the injection flow velocity of the injection pump reaches a limit value and the physical characteristic data exceeds the reference value range;

When the injection flow velocity of the injection pump reaches the limit value, but the temperature value of the RF operating object still does not fall back within the reference value range, the temperature value of the RF operating object cannot be further controlled by adjusting the injection flow velocity, and thus a RF output power is adjusted to further adjust the temperature value of the RF operating object.

The power adjustment value of the RF host is calculated according to electrical parameters of the electrodes of the multi-electrode RF probe, which is the minimum impedance value.

Step S304, adjusting the RF output power to a second power according to the first power and the power adjustment value and then outputting the second power.

In the embodiments of the present invention, the injection pump is first controlled to inject the liquid into the RF operating object according to the preset injection flow velocity when the physical characteristic data exceeds the reference value range, so as to adjust the physical characteristic data to be within the reference value range. If the physical characteristic data still exceeds the reference value range when the injection flow velocity reaches the limit value, the power adjustment value of the RF host is then calculated according to the electrical parameters of the electrodes of the multi-electrode RF probe, and the output power is adjusted according to the power adjustment value. By means of adding the electrical parameters of the electrodes into the calculation, an accuracy of power control adjustment can be improved, and an effectiveness of RF operation can be improved.

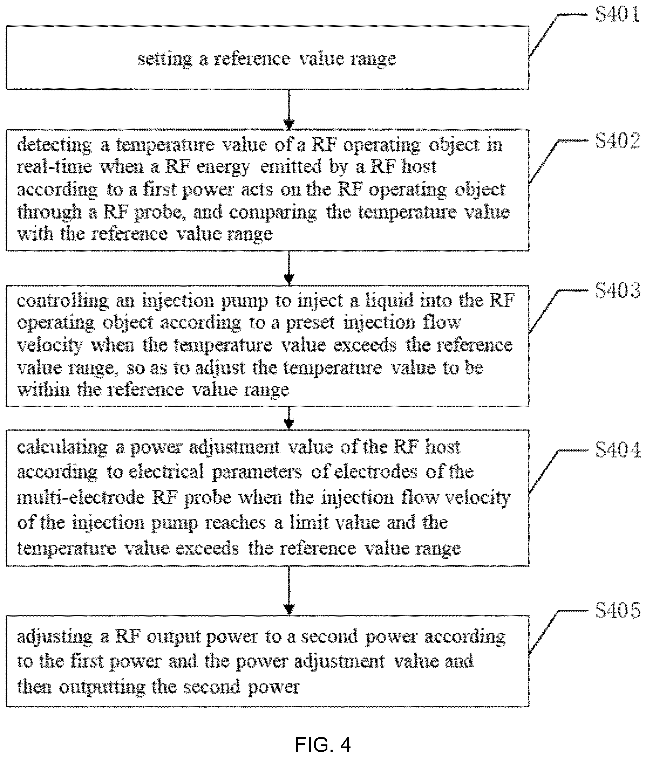

Referring to FIG. 4, which is a flowchart of a RF operation protection method provided by another embodiment of the present invention. The method may be applied to the RF host as shown in FIG. 1. As shown in FIG. 4, the method specifically includes:

Step S401, setting a reference value range;
A setting method may be that: the RF host responds to user instructions, displays setting limits of the reference value range on a display interface of the RF host, obtains the minimum and maximum values of the user input reference values, and generates the reference value range for the temperature value of the operating object in the RF operation after getting a generating instruction.

The setting method may also be that: the reference value range is acquired from the historical RF operation data stored in the database of the server connected to the RF host, wherein the server stores the historical RF operation data of all RF hosts connected to the server. Through a preset data analysis method, the reference value range may be obtained. For example, for RF operations with the same operating object and operating content, an average temperature value may be taken as a middle value of the reference value range; or, an average of the maximum temperature values may be taken as the maximum value of the reference value range.

Step S402: detecting a temperature value of a RF operating object in real-time when a RF energy emitted by a RF host according to a first power acts on the RF operating object through a RF probe, and comparing the temperature value with the reference value range;
The multi-electrode RF probe includes multiple electrodes, which act on the designated position of the RF operating object simultaneously during the RF operation. There is no limit to the number of multiple electrodes, which may be 3, 4, 6, and etc., depending on the purpose of the RF operation, the nature of the RF operation object, and the characteristics of the designated position.

The physical characteristic data may be the temperature value of the RF operating object.

When the multi-electrode RF probe performs the RF operation on the RF operating object, the physical characteristic data of the RF operating object will change. The physical characteristic data is compared with the preset reference value range to determine whether the physical characteristic data exceeds the reference value range, for example, determine whether the temperature value of the RF operating object exceeds a preset temperature reference value range.

Step S403: controlling an injection pump to inject a liquid into the RF operating object according to a preset injection flow velocity when the temperature value exceeds the reference value range, so as to adjust the temperature value to be within the reference value range;
The reference value range is a numerical range with the minimum and maximum values. The physical characteristic data exceeds the reference value range, that is, it is greater than the maximum value of the reference value range, or it is less than the minimum value of the reference value range.

When a control system of the RF host determines that the physical characteristic data of the RF operating object exceeds the reference value range, it sends a control instruction immediately or after a preset waiting time (such as 3 seconds) to a control system of the injection pump to control the injection pump to inject the liquid into the RF operating object according to the preset injection flow velocity, so as to adjust the physical characteristic data to be within the reference value range. The preset injection flow velocity is greater or less than the current injection flow velocity. Increasing the injection flow velocity can decrease the temperature value of the RF operating object, while decreasing the injection flow velocity can increase the temperature value of the RF operating object.

Step S404: calculating a power adjustment value of the RF host according to electrical parameters of electrodes of the multi-electrode RF probe when the injection flow velocity of the injection pump reaches a limit value and the temperature value exceeds the reference value range;
When the injection flow velocity of the injection pump reaches the limit value, but the temperature value of the RF operating object still does not fall back within the reference value range, the temperature value of the RF operating object cannot be further controlled by adjusting the injection flow velocity, and thus a RF output power is adjusted to further adjust the temperature value of the RF operating object.

By means of a preset PID algorithm, a total power that needs to be set is determined according to the minimum impedance value of the electrodes of the multi-electrode RF probe. A current total power of the multi-electrode RF probe is detected in real-time. According to the total power that needs to be set and the total power in real-time, the power adjustment value of the RF host is calculated through the preset PID algorithm.

Specifically, the impedance values of the multiple electrodes of the multi-electrode RF probe are detected, and a single electrode with the minimum impedance value is determined thereby. The total power that needs to be set is calculated according to the power of the single electrode, which is an upper limit of the power that the electrodes may reach.

According to the power calculation formula $P = U^2 / R$, the electrodes have the same voltage at the RF operating position as the electrodes of the multi-electrode RF probe are connected to the same voltage output, and the power of each electrode is determined by its impedance R, the smaller R, the greater P. The power of each electrode is limited to the set total power, which may be equal to but cannot exceed the set total power.

Specifically, the total power that needs to be set currently is calculated according to the impedance of the electrode.

According to $P = U^2 / R$, a relationship between the power $P_{lim}$ of the single electrode with the lowest impedance value and the power $P_n$ of other electrodes is:

$$\frac{P_{lim}}{P_n} = \frac{U^2/R_{lim}}{U^2/R_n} = \frac{R_n}{R_{lim}}, \text{ that is, } P_n = \frac{P_{lim}R_{lim}}{R_n}.$$

$P_{lim}$ is the power of the known electrode with the minimum impedance value. According to $R_{lim}$ and the impedance values $R_n$ of other electrodes, $P_n$ corresponding to each electrode may be obtained. A calculation formula for the total

$$P = \sum_{i=1}^{n} P_i$$

power P that needs to be set is:

According to the PID algorithm, an increment of the total power $\Delta P$ may be obtained based on the current total power measured in real-time and the total power P that needs to be set, wherein formulas for the PID algorithm are as follows:

$$\text{Formula 1:} \quad u(k) = K_P\{err(k) + \frac{T}{T_I}\sum_{j=0}^{k}err(j) + \frac{T_D}{T}[err(k) - err(k-1)]\};$$

or,

$$\text{Formula 2:} \quad u(k) = K_P err(k) + K_I\sum_{j=0}^{k}err(j) + K_D[err(k) - err(k-1)];$$

$$K_I = K_P\frac{T}{T_I}, \quad K_D = K_P\frac{T_D}{T}$$

wherein $K_P$, represent proportional coefficient, integral coefficient and differential coefficient of the PID algorithm, respectively, $T$ represents the sampling time, $T_I$ represents the integral time (also known as the integral coefficient), $T_D$ represents the differential time (also known as the differential coefficient), $err(k)$ represents a difference between the total power P that needs to be set and the total power in real-time, and $u(k)$ represents the output power.

Using an incremental PID algorithm, $\Delta P = u(k)-u(k-1)$, and according to the above Formula 2, it is obtained that:

$$\Delta P = K_P[err(k) - err(k-1)] + K_I err(k) + K_D[err(k) - 2err(k-1) + err(k-2)]$$

Step S405: adjusting a RF output power to a second power according to the first power and the power adjustment value and then outputting the second power.

An adjustment amount of output is calculated according to $\Delta P$, which has a one-to-one correspondence with $\Delta P$. Since the power adjustment is achieved by controlling a voltage signal of a power board, the output voltage corresponds to the input digital signal of an A/D converter, the adjustment amount actually corresponds to this digital signal, and the corresponding relationship is made between the output and $\Delta P$. For example, if the output is 1, it means that the corresponding power increment is 0.1w. Therefore, controlling of $\Delta P$ may be achieved according to the corresponding relationship.

The second power is obtained according to the first power and the power increment $\Delta P$. When $\Delta P$ is a negative value, the power increment $\Delta P$ represents a decrease to the RF output power to lower the temperature. Conversely, when $\Delta P$ is a positive value, it represents an increase to the RF output power to increase the temperature.

The RF output power is adjusted to the second power and then outputs.

In the embodiments of the present invention, the injection pump is first controlled to inject the liquid into the RF operating object according to the preset injection flow velocity when the temperature value of the RF operating object exceeds the reference value range, so as to adjust the temperature value of the RF operating object back within the reference value range. If the temperature value still exceeds the reference value range when the injection flow velocity reaches the limit value, the single electrode with the minimum impedance value is determined among the electrodes of the multi-electrode RF probe, and the total power that needs to be set is calculated according to the impedance value of the single electrode. Further, the power adjustment value of the RF host is calculated through the PID algorithm according to the total power that needs to be set and the detected total power of the multi-electrode RF probe in real-time, which greatly improves the setting accuracy of the output power and achieves better RF operation results.

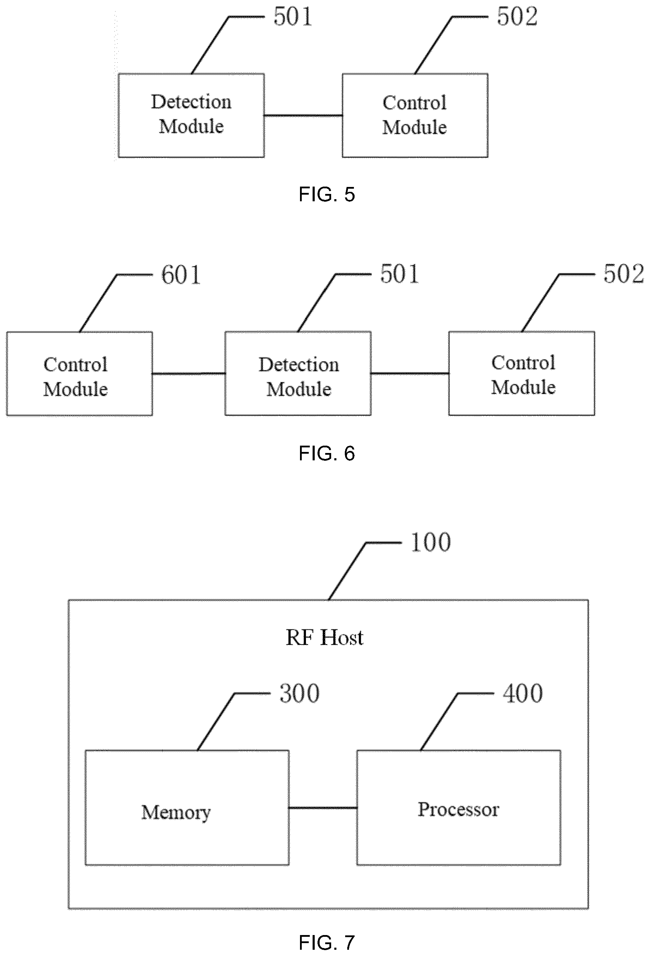

Refer to FIG. 5, which is a schematic view of a RF host provided by an embodiment of the present invention. For ease of description, only the parts related to the embodiments of the present invention are shown. The RF host is the RF host that executes the power adjustment method for multi-electrode RF probe in the above embodiments, and includes:

a detection module 501, configured for controlling an injection pump to inject a liquid into the RF operating object according to a preset injection flow velocity when the physical characteristic data exceeds the reference value range, so as to adjust the physical characteristic data to be within the reference value range;

a control module 502, configured for controlling an injection pump to inject a liquid into the RF operating object according to a preset injection flow velocity when the physical characteristic data exceeds the reference value range, so as to adjust the physical characteristic data to be within the reference value range;

the control module 502 further configured for calculating a power adjustment value of the RF host according to electrical parameters of electrodes of the multi-electrode RF probe when the injection flow velocity of the injection pump reaches a limit value and the physical characteristic data exceeds the reference value range;

the control module further 502 configured for adjusting a RF output power to a second power according to the first power and the power adjustment value and then outputting the second power.

In the above modules, the control module first controls the injection pump to inject the liquid into the RF operating object according to the preset injection flow velocity when the detection module detects that the physical characteristic data of the RF operating object exceeds the reference value range, so as to adjust the physical characteristic data to be within the reference value range. If the physical characteristic data still exceeds the reference value range when the injection flow velocity reaches the limit value, the control module calculates the power adjustment value of the RF host according to the electrical parameters of the electrodes of the multi-electrode RF probe and the output power is adjusted according to the adjustment value. By means of adding the electrical parameters of the electrodes into the calculation, an accuracy of adjusting of the output power may be improved, and an effectiveness of the RF operation may be improved.

Further, the physical characteristic data of the RF operating object includes a temperature value of the RF operating object.

Further, referring to FIG. 6, the RF host further includes a setting module 601.

The setting module 601 is configured for acquiring the maximum and minimum values of temperature values input on an input interface, and generating a preset reference value range according to the maximum and minimum values of the temperature values; or acquiring historical data of a RF operation from a database of a server and getting the preset reference value range according to temperature values of the operating object in the historical data.

The control module 502 is further used to control the injection pump to inject the liquid into the RF operating object according to a preset first injection flow velocity when the temperature value is greater than the maximum value of the reference value range, so as to adjust the temperature value to be less than the maximum value of the reference value range, wherein the first injection flow velocity is greater than the current injection flow velocity; or control the injection pump to inject the liquid into the RF operating object according to a preset second injection flow velocity when the temperature value is less than the minimum value of the reference value range, so as to adjust the temperature value to be greater than the minimum value of the reference value range, wherein the second injection flow velocity is less than the current injection flow velocity.

Further, the control module 502 is also used to determine a total power that needs to be set according to the minimum impedance value of the electrodes of the multi-electrode RF probe; detect a current total power of the multi-electrode RF probe in real-time, and calculate the power adjustment value of the RF host through a preset PID algorithm according to the total power that needs to be set and the total power in real-time.

Further, the control module 502 is also used to determine a single electrode with the minimum impedance value among the electrodes of the multi-electrode RF probe; calculate a power of each other electrodes according to an impedance value of the single electrode, a preset power of the single electrode and an impedance value of each other electrodes except for the single electrode of the multi-electrode RF probe, and take a sum of the power of the multiple electrodes as the total power that needs to be set

A calculation formula for calculating the power adjustment value ΔP of the RF host through the PID algorithm is as follows:

$$\Delta P = K_P[err(k) - err(k-1)] + K_I err(k) + K_D[err(k) - 2err(k-1) + err(k-2)]$$

$$K_I = K_P \frac{T}{T_I}, \quad K_D = K_P \frac{T_D}{T}$$

Wherein $K_P$, represent proportional coefficient, integral coefficient and differential coefficient of the PID algorithm, respectively, $T$ represents the sampling time, $T_I$ represents the integral time, $T_D$ represents the differential time, and $err(k)$ represents a difference between the total power that needs to be set and the total power in real-time.

Other calculation details please refer to the relevant description of the embodiments of the power adjustment method for multi-electrode RF probe described above.

Further, the control module 502 is also used to subtract the power adjustment value from the first power to obtain the second power when the temperature value of the operating object is higher than the maximum value of the reference value range, and adjust the RF output power to the second power and then outputting the second power.

In the embodiments of the present invention, the injection pump is first controlled to inject the liquid into the RF operating object according to the preset injection flow velocity when the temperature value of the RF operating object exceeds the reference value range, so as to adjust the temperature value of the RF operating object back within the reference value range. If the temperature value still exceeds the reference value range when the injection flow velocity reaches the limit value, the single electrode with the minimum impedance value is determined among the electrodes of the multi-electrode RF probe, and the total power that needs to be set is calculated according to the impedance value of the single electrode. Furthermore, the power adjustment value of the RF host is calculated through the PID algorithm according to the total power that needs to be set and the detected total power of the multi-electrode RF probe in real-time, which greatly improves the setting accuracy of output power and achieves better RF operation results.

Further, as shown in FIG. 7, an embodiment of the present invention further provides a RF host that includes a memory 300 and a processor 400. The processor 400 may be a central processor of the RF host in the above embodiments. The storage 300 may be, such as a hard disk drive memory, a non-volatile memory (such as a flash memory or other electronically programmable restricted deletion memory configured to form a solid-state drive), and a volatile memory (for example, a static or dynamic random access memory and the like), and the like, which is not limited in the embodiments of the present invention.

The memory 300 stores executable program codes thereon, and the processor 400 is electrically coupled to the memory 300, calling the executable program codes stored on the memory, and executing the power adjustment method for multi-electrode RF probe as described above.

Further, an embodiment of the present application further provides a computer-readable storage medium. The computer-readable storage medium may be set in the RF host of each of the foregoing embodiments, or may be the memory 300 of the embodiment as shown in FIG. 7. A computer program is stored on the computer-readable storage medium, and is executed by the processor to realize the power adjustment method for multi-electrode RF probe described in the foregoing embodiments as shown in FIG. 3 and FIG. 4. Further, the computer-storable storage medium may further be a U disk, a mobile hard disk, a read-only memory (ROM), a RAM, a magnetic disk or an optical disk, and other various media that may store the program code.

In the above-mentioned embodiments, descriptions of the embodiments have particular emphasis respectively. For parts that are not described in detail in a certain embodiment, reference may be made to related descriptions of other embodiments.

The above is a description of the power adjustment method for multi-electrode RF probe and the RF host according to the present invention. For those skilled in the art, according to the ideas of the embodiments of the present invention, changes may be made to specific implementations and application scopes. In summary, the content of this specification should not be construed as a limitation on the present invention.

**Claims**

1. A power adjustment method for multi-electrode radio frequency probe, comprising:

   detecting physical characteristic data of a radio frequency operating object in real-time when a radio frequency energy emitted by a radio frequency host according to a first power acts on the radio frequency operating object through a multi-electrode radio frequency probe, and comparing the physical characteristic data with a preset reference value range;

   controlling an injection pump to inject a liquid into the radio frequency operating object according to a preset injection flow velocity when the physical characteristic data exceeds the reference value range, so as to adjust the physical characteristic data to be within the reference value range;

   calculating a power adjustment value of the radio frequency host according to electrical parameters of electrodes of the multi-electrode radio frequency probe when the injection flow velocity of the injection pump reaches a limit value and the physical characteristic data exceeds the reference value range;

   adjusting a radio frequency output power to a second power according to the first power and the power adjustment value and then outputting the second power.

2. The method according to claim 1, wherein the physical characteristic data of the radio frequency operating object comprises a temperature value of the radio frequency operating object.

3. The method according to claim 2, wherein before the step of detecting the physical characteristic data of the radio frequency operating object in real-time when the radio frequency energy emitted by the radio frequency host according to the first power acts on the radio frequency operating object through the multi-electrode radio frequency probe, the method further comprises:

   acquiring the maximum and minimum values of temperature values input on an input interface, and generating the preset reference value range according to the maximum and minimum values of the temperature values; or acquiring historical data of a radio frequency operation from a database of a server and getting the preset reference value range according to temperature values of the operating object in the historical data.

4. The method according to claim 3, wherein the step of controlling the injection pump to inject the liquid into the radio frequency operating object according to the preset injection flow velocity when the physical characteristic data exceeds the reference value range, so as to adjust the physical characteristic data to be within the reference value range comprises:

   controlling the injection pump to inject the liquid into the radio frequency operating object according to a preset first injection flow velocity when the temperature value is greater than the maximum value of the reference value range, so as to adjust the temperature value to be less than the maximum value of the reference value range, wherein the first injection flow velocity is greater than the current injection flow velocity;

   controlling the injection pump to inject the liquid into the radio frequency operating object according to a preset second injection flow velocity when the temperature value is less than the minimum value of the reference value range, so as to adjust the temperature value to be greater than the minimum value of the reference value range, wherein the second injection flow velocity is less than the current injection flow velocity.

5. The method according to any one of claims 1-4, wherein the step of calculating the power adjustment value of the radio frequency host according to the electrical parameters of the electrodes of the multi-electrode radio frequency probe comprises:

   determining a total power that needs to be set according to the minimum impedance value of the electrodes of the multi-electrode radio frequency probe;

   detecting a current total power of the multi-electrode radio frequency probe in real-time, and calculating the power adjustment value of the radio frequency host through a preset PID algorithm according to the total power that needs to be set and the total power in real-time.

6. The method according to claim 5, wherein the step of determining the total power that needs to be set according to the minimum impedance value of the electrodes of the multi-electrode radio frequency probe comprises:

   determining a single electrode with the minimum impedance value among the electrodes of the multi-electrode radio frequency probe;

calculating a power of each other electrodes according to an impedance value of the single electrode, a preset power of the single electrode and an impedance value of each other electrodes except for the single electrode of the multi-electrode radio frequency probe, and taking a sum of the power of the multiple electrodes as the total power that needs to be set.

7. The method according to claim 6, wherein the step of calculating the power adjustment value of the radio frequency host through the preset PID algorithm comprises:

a computing formula for calculating the power adjustment value $\Delta P$ of the radio frequency host through the PID algorithm as follows:

$$\Delta P = K_P[err(k) - err(k-1)] + K_I err(k) + K_D[err(k) - 2err(k-1) + err(k-2)],$$

$$K_I = K_P \frac{T}{T_I}, \quad K_D = K_P \frac{T_D}{T}$$

Wherein $K_P$, represent proportional coefficient, integral coefficient and differential coefficient of the PID algorithm, respectively, $T$ represents the sampling time, $T_I$ represents the integral time, $T_D$ represents the differential time, and $err(k)$ represents a difference between the total power that needs to be set and the total power in real-time.

8. The method according to claim 7, wherein the step of adjusting the radio frequency output power to the second power according to the first power and the power adjustment value and then outputting the second power comprises: subtracting the power adjustment value from the first power to obtain the second power when the temperature value of the operating object is higher than the maximum value of the reference value range, and adjusting the radio frequency output power to the second power and then outputting the second power.

9. A radio frequency host comprising:

a detection module, configured for controlling an injection pump to inject a liquid into the radio frequency operating object according to a preset injection flow velocity when the physical characteristic data exceeds the reference value range, so as to adjust the physical characteristic data to be within the reference value range;
a control module, configured for controlling an injection pump to inject a liquid into the radio frequency operating object according to a preset injection flow velocity when the physical characteristic data exceeds the reference value range, so as to adjust the physical characteristic data to be within the reference value range;
the control module further configured for calculating a power adjustment value of the radio frequency host according to electrical parameters of electrodes of the multi-electrode radio frequency probe when the injection flow velocity of the injection pump reaches a limit value and the physical characteristic data exceeds the reference value range;
the control module further configured for adjusting a radio frequency output power to a second power according to the first power and the power adjustment value and then outputting the second power.

10. A radio frequency host comprising a memory and a processor, the memory storing executable program codes thereon, the processor being electrically coupled to the memory, calling the executable program codes stored on the memory and executing the power adjustment method for multi-electrode radio frequency probe defined in any one of claims 1-8.

FIG. 1

FIG. 2

detecting physical characteristic data of a RF operating object in real-time when a RF energy emitted by a RF host according to a first power acts on the RF operating object through a RF probe, and comparing the physical characteristic data with a preset reference value range ⎯ S301

controlling an injection pump to inject a liquid into the RF operating object according to a preset injection flow velocity when the physical characteristic data exceeds the reference value range, so as to adjust the physical characteristic data to be within the reference value range ⎯ S302

calculating a power adjustment value of the RF host according to electrical parameters of electrodes of the multi-electrode RF probe when the injection flow velocity of the injection pump reaches a limit value and the physical characteristic data exceeds the reference value range ⎯ S303

adjusting a RF output power to a second power according to the first power and the power adjustment value and then outputting the second power ⎯ S304

FIG. 3

```
                                                              S401
┌──────────────────────────────────────────────────────┐
│            setting a reference value range             │
└──────────────────────────────────────────────────────┘
                          │
                          ▼
                                                              S402
┌──────────────────────────────────────────────────────┐
│  detecting a temperature value of a RF operating       │
│  object in real-time when a RF energy emitted by a RF  │
│  host according to a first power acts on the RF        │
│  operating object through a RF probe, and comparing    │
│  the temperature value with the reference value range  │
└──────────────────────────────────────────────────────┘
                          │
                          ▼
                                                              S403
┌──────────────────────────────────────────────────────┐
│  controlling an injection pump to inject a liquid into │
│  the RF operating object according to a preset         │
│  injection flow velocity when the temperature value    │
│  exceeds the reference value range, so as to adjust    │
│  the temperature value to be within the reference      │
│  value range                                           │
└──────────────────────────────────────────────────────┘
                          │
                          ▼
                                                              S404
┌──────────────────────────────────────────────────────┐
│  calculating a power adjustment value of the RF host   │
│  according to electrical parameters of electrodes of   │
│  the multi-electrode RF probe when the injection flow  │
│  velocity of the injection pump reaches a limit value  │
│  and the temperature value exceeds the reference value │
│  range                                                 │
└──────────────────────────────────────────────────────┘
                          │
                          ▼
                                                              S405
┌──────────────────────────────────────────────────────┐
│  adjusting a RF output power to a second power         │
│  according to the first power and the power adjustment │
│  value and then outputting the second power            │
└──────────────────────────────────────────────────────┘
```

FIG. 4

501

502

Detection
Module

Control
Module

FIG. 5

601

501

502

Control
Module

Detection
Module

Control
Module

FIG. 6

100

RF Host

300

400

Memory

Processor

FIG. 7

## INTERNATIONAL SEARCH REPORT

| International application No. |
| --- |
| **PCT/CN2021/142752** |

**A. CLASSIFICATION OF SUBJECT MATTER**

A61B 18/12(2006.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNTXT, WPABSC, ENTXTC, CNKI: 电极, 射频, 功率, 范围, 阈值, 比较, 对比, 注射, 输送, 液体, 流体, 介质, 温度, 电参数, 阻抗 VEN, WPABS, ENTXT: electrode?, RF, radio-frequency, power, zone, threshold, range, limit, than, compare, inject, delivery, convey, liquid, fluid, medium, temperature, parameter, resistance, impedance

**C. DOCUMENTS CONSIDERED TO BE RELEVANT**

| Category* | Citation of document, with indication, where appropriate, of the relevant passages | Relevant to claim No. |
| --- | --- | --- |
| PX | CN 113143443 A (HANGZHOU KUNBO BIOTECHNOLOGY CO., LTD.) 23 July 2021 (2021-07-23)<br>claims 1-10 | 1-10 |
| PY | CN 112807071 A (HANGZHOU KUNBO BIOTECHNOLOGY CO., LTD.) 18 May 2021 (2021-05-18)<br>description, paragraphs 16-85, and figures 1-5 | 1-10 |
| PY | CN 112773497 A (HANGZHOU KUNBO BIOTECHNOLOGY CO., LTD.) 11 May 2021 (2021-05-11)<br>description, paragraphs 16-91, and figures 1-5 | 1-10 |
| Y | CN 110897710 A (HANGZHOU KUNBO BIOTECHNOLOGY CO., LTD.) 24 March 2020 (2020-03-24)<br>description paragraphs 24-146 | 1-10 |
| Y | CN 106580465 A (BIOSENSE WEBSTER (ISRAEL), LTD.) 26 April 2017 (2017-04-26)<br>description paragraphs 38-69, figure 7 | 1-5, 9-10 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **25 February 2022** | **28 March 2022** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2021/142752** |

**C. DOCUMENTS CONSIDERED TO BE RELEVANT**

| Category* | Citation of document, with indication, where appropriate, of the relevant passages | Relevant to claim No. |
| --- | --- | --- |
| Y | CN 202942209 U (SYNAPTIC MEDICAL TECHNOLOGY (BEIJING) CO., LTD.) 22 May 2013 (2013-05-22)<br>    description, paragraphs 10-14, figure 1 | 1-5, 9-10 |
| Y | CN 107981928 A (BEIJING BHKY MEDICAL INSTRUMENT CO., LTD.) 04 May 2018 (2018-05-04)<br>    description, paragraphs 21-33, and figures 1-2 | 1-5, 9-10 |
| Y | US 6059780 A (R. ITA MEDICAL SYSTEMS, INC.) 09 May 2000 (2000-05-09)<br>    description, column 3 line 65 to column 7 line 40, figures 1-10 | 1-5, 9-10 |
| A | CN 1631327 A (SHANGHAI JIAO TONG UNIVERSITY) 29 June 2005 (2005-06-29)<br>    entire document | 1-10 |
| A | CN 200942123 Y (MEDSPHERE INTERNATIONAL, INC.) 05 September 2007 (2007-09-05)<br>    entire document | 1-10 |

Form PCT/ISA/210 (second sheet) (January 2015)

## INTERNATIONAL SEARCH REPORT
### Information on patent family members

International application No.

**PCT/CN2021/142752**

| Patent document cited in search report | | | Publication date (day/month/year) | Patent family member(s) | | | Publication date (day/month/year) |
|---|---|---|---|---|---|---|---|
| CN | 113143443 | A | 23 July 2021 | None | | | |
| CN | 112807071 | A | 18 May 2021 | CN | 112807071 | B | 04 March 2022 |
| CN | 112773497 | A | 11 May 2021 | None | | | |
| CN | 110897710 | A | 24 March 2020 | CN | 110897710 | B | 31 August 2021 |
| CN | 106580465 | A | 26 April 2017 | CA | 2945428 | A1 | 16 April 2017 |
| | | | | JP | 2017077463 | A | 27 April 2017 |
| | | | | JP | 6843579 | B2 | 17 March 2021 |
| | | | | RU | 2016140227 | A | 16 April 2018 |
| | | | | RU | 2016140227 | A3 | 13 February 2020 |
| | | | | AU | 2001234997 | A1 | 01 November 2001 |
| | | | | IL | 248142 | D0 | 31 January 2017 |
| | | | | IL | 248142 | A | 27 February 2020 |
| | | | | US | 2017105783 | A1 | 20 April 2017 |
| | | | | US | 10285751 | B2 | 14 May 2019 |
| | | | | EP | 3158961 | A1 | 26 April 2017 |
| | | | | EP | 3158961 | B1 | 26 December 2018 |
| | | | | ES | 2711750 | T3 | 07 May 2019 |
| | | | | AU | 2016234997 | A1 | 04 May 2017 |
| | | | | IN | 201614034003 | A | 26 May 2017 |
| | | | | CN | 106580465 | B | 20 April 2021 |
| CN | 202942209 | U | 22 May 2013 | None | | | |
| CN | 107981928 | A | 04 May 2018 | None | | | |
| US | 6059780 | A | 09 May 2000 | US | 6500175 | B1 | 31 December 2002 |
| CN | 1631327 | A | 29 June 2005 | CN | 1278654 | C | 11 October 2006 |
| CN | 200942123 | Y | 05 September 2007 | None | | | |

Form PCT/ISA/210 (patent family annex) (January 2015)